Europäisches Patentamt

European Patent Office

Office européen des brevets

(11) Veröffentlichungsnummer: **0 345 548**
**A1**

(12) # EUROPÄISCHE PATENTANMELDUNG

(21) Anmeldenummer: **89109435.1**

(22) Anmeldetag: **24.05.89**

(51) Int. Cl.⁴: **A61B 6/10**

(30) Priorität: **08.06.88 DE 8807462 U**

(43) Veröffentlichungstag der Anmeldung:
**13.12.89 Patentblatt 89/50**

(84) Benannte Vertragsstaaten:
**BE DE FR GB IT**

(71) Anmelder: **Siemens Aktiengesellschaft**
**Wittelsbacherplatz 2**
**D-8000 München 2(DE)**

(72) Erfinder: **Hüttenrauch, Gerd, Dipl.-Ing.**
**Sudetenstrasse 14**
**D-8525 Uttenreuth(DE)**
Erfinder: **Schelchshorn, Herbert**
**Würzburger Ring 7**
**D-8520 Erlangen(DE)**

(54) **Strahlenschutzvorrichtung.**

(57) Wie bekannt belastet eine Strahlenschutzschürze wegen ihres hohen Eigengewicht die Untersucherungsperson, ohne perstrahlenschutz zu bieten. Auch
Bleivorhänge als Strahlenschutz am Röntgengerät
werden von der Untersuchungsperson und den Patienten als lästig empfunden. Eine Strahlenschutzvorrichtung (1) besitzt eine Röntgenstrahlen absorbierende Strahlenschutzwand, einen die Strahlenschutzwand tragenden, frei verfahrbaren Wagen (2)
und eine höhenverstellbare Halterung (4) für zwei
Wandelemente (5, 6), wobei das erste Wandelement
(5) unterhalb des zweiten, durchsichtigen Wandelementes (6) angeordnet ist und wobei das zweite
Wandelement (6) um eine horizontale Achse (7)
schwenkbar ist.

EP 0 345 548 A1

## Strahlenschutzvorrichtung

Die Erfindung betrifft eine Strahlenschutzvorrichtung für eine einer Strahlung ausgesetzte Untersuchungsperson.

Bei der röntgenologischen Untersuchung eines Patienten muß vermieden werden, daß der Röntgenologe einer unzulässig hohen Strahlenbelastung ausgesetzt ist, insbesondere dann, wenn es sich um patientennahe Untersuchungen handelt, die an Röntgengeräten mit Obertischstrahlern durchgeführt werden.

Zum Strahlenschutz verwendet der Röntgenologe bei patientennahen Untersuchungen einen Bleigummimantel. Dieser Bleigummimantel ist jedoch durch das hohe Eigengewicht insbesondere bei längeren Untersuchungen oder Operationen für den Röntgenologen körperlich sehr belastend und durch die geringe Flexibilität störend. Der Kopf und insbesondere die Augen des Röntgenologen werden nicht geschützt.

Eine weitere Strahlenschutzmaßnahme besteht darin, Bleigummivorhänge entweder an der Raumdecke oder am Untersuchungsgerät zu befestigen. Diese Bleigummivorhänge behindern allerdings den Röntgenologen während seiner Arbeit und berühren bzw. belästigen den Patienten.

Aufgabe der Erfindung ist es daher, eine Strahlenschutzvorrichtung der eingangs genannten Art so auszubilden, daß sie für eine Untersuchungsperson einen möglichst guten Ganzkörperstrahlenschutz bietet und die Untersuchungsperson weder bei der Arbeit behindert noch körperlich belastet.

Die Aufgabe wird erfindungsgemäß durch folgende Merkmale gelöst:

Eine Strahlenschutzvorrichtung besitzt einen, eine Strahlenschutzwand tragenden Wagen, der frei verfahrbar ist und eine Halterung für zwei Wandelemente, wobei die Halterung höhenverstellbar auf dem Wagen montiert ist, wobei das erste Wandelement unterhalb des zweiten, durchsichtigen Wandelementes angeordnet ist und wobei das zweite Wandelemente um eine horizontale Achse schwenkbar ist.

Vorteil der Erfindung ist, daß mit der erfindungsgemäßen Strahlenschutzvorrichtung der Kopf und insbesondere die Augen der Untersuchungsperson strahlengeschützt sind und daß die Untersuchungsperson durch das durchsichtige Wandelement freie Sicht zum Arbeitsbereich hat.

Durch die erfindungsgemäße Strahlenschutzvorrichtung wird vorteilhaft erreicht, daß die Untersuchungsperson vom Tragen einer schweren Bleigummischürze, wie es beim Stand der Technik üblich ist, befreit ist, was sich insbesondere bei längeren Operationen vorteilhaft auswirkt. Außerdem kann der Strahlenschutz für die Untersuchungsperson durch die Verwendung von einem Material mit einer größeren Wandstärke verbessert werden, ohne daß die Untersuchungsperson durch ein höheres Gewicht belastet wird.

Die Unteransprüche stellen vorteilhafte Ausführungsformen der Erfindung dar.

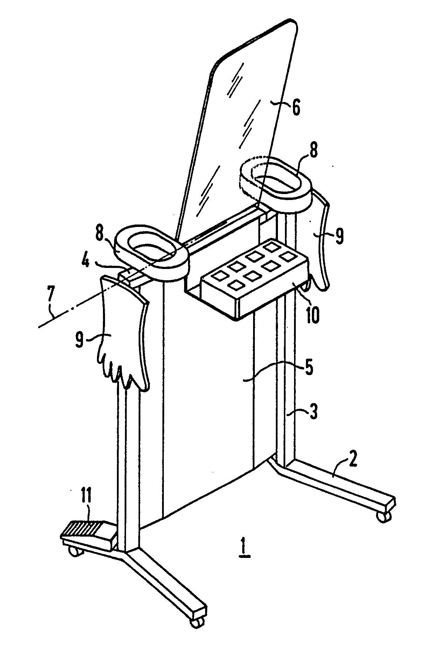

Ein Ausführungsbeispiel der Erfindung wird nachfolgend anhand der Figur beschrieben.

Nach der Figur besitzt eine Strahlenschutzvorrichtung 1 ein Fahrgestell 2, an dem über ein Stativ 3 eine Halterung 4 montiert ist. Die Halterung 4 ist als Balken annähernd parallel zum Boden (horizontal) ausgerichtet und haltert zwei Wandelemente 5, 6 zum Absorbieren von Röntgenstrahlung. Ein erstes, beispielsweise aus Bleigummi bestehendes Wandelement 5 reicht von der Halterung 4 bis kurz vor das Bodenniveau. Ein zweites Wandelement 6 ist dem ersten Wandelement 5 gegenüberliegend an der Halterung 4 befestigt und ist aus einem durchsichtigen Material hergestellt. Vorzugsweise kann das Wandelement 6 aus Bleiglas hergestellt sein. Die Höhe der Strahlenschutzvorrichtung 1 ist so bemessen, daß sich die Halterung 4 im Bereich des Oberkörpers einer durchschnittlichen Untersuchungsperson, beispielsweise eines Röntgenologen, befindet und daß das Wandelement 6 die Kopfhöhe überragt. Damit sind der Kopf und insbesondere die Augen strahlengeschützt, wenn der Röntgenologe beispielsweise bei einer Durchleuchtung hinter der Strahlenschutzvorrichtung 1 steht. Die Breite des Wandelementes 6 ist so bemessen, daß mindestens der Oberkörper überdeckt wird. Dabei sollte die Bewegungsfreiheit der Arme in Richtung zum Arbeitsbereich nicht eingeschränkt sein. Die Breite des Wandelementes 6 bzw. eines Bereiches des Wandelementes 6 ist also so gewählt, daß der Röntgenologe das Wandelement 6 bzw. den Bereich des Wandelementes 6 bequem umfassen kann und damit freien Zugang zum Arbeitsbereich hat. Es können aber auch Aussparungen für die Arme des Röntgenologen im Wandelement 6 vorgesehen sein. Das zweite Wandelement 6 kann aber auch als Fenster in eine Halterung, die über die Kopfhöhe einer durchschnittlichen Untersuchungsperson reicht, eingebracht sein.

Um die Strahlenschutzvorrichtung 1 an die individuellen Bedürfnisse einer Untersuchungsperson anpassen zu können, ist die Halterung 4 über das Stativ 3 höhenverstellbar. Zudem ist das Wandelement 6 um die Längsachse 7 der Halterung 4 schwenkbar gelagert. Hierdurch kann die Strahlenschutzvorrichtung 1 zum optimalen Strahlenschutz individuell auf die Untersuchungsperson und die jeweiligen Bedingungen im Untersuchungsbereich optimal eingestellt werden.

Bei therapeutischen Eingriffen am Patienten unter Durchleuchtung ist es besonders vorteilhaft, wenn die Untersuchungsperson, beispielsweise ein Chirurg, seine Ellenbogen in Stützschalen 8 abstützen kann. Hierdurch können die Beine und der Oberkörper des Chirurgen entlastet werden mit einem erleichterten Arbeiten im Operationsgebiet. Diese Stützschalen 8 sind seitlich neben dem Wandelement 6 auf der Halterung 4 angebracht und können, da sie höhenverstellbar sind, ebenfalls auf die individuellen Bedürfnisse der Untersuchungsperson eingestellt werden. Die Stützschalen 8 können aber auch auf gesonderten Stützen höhenverstellbar gelagert sein.

Ein weiterer Vorteil der Stützschalen 8 ist darin zu sehen, daß die Strahlenschutzvorrichtung 1 über die abgestützten Ellenbogen leicht im Untersuchungsbereich verfahren werden kann. Dies ist insbesondere deshalb vorteilhaft, weil die Hände der Untersuchungsperson beim Verfahren der Strahlenschutzvorrichtung 1 steril bleiben.

Wenn es erforderlich sein sollte, daß die Untersuchungsperson während einer Durchleuchtung im Operationsgebiet hantieren muß, sind hierzu Bleihandschuhe 9 notwendig. Diese Bleihandschuhe 9 sind in Halterungen (nicht gezeigt) jeweils seitlich im Bereich der Halterung 4 gehalten. Die Halterungen sind vorteilhafterweise so beschaffen, daß sie eine Einhandfunktion beim Überstreifen der Bleihandschuhe 9 erlauben.

An der dem Untersuchungsbereich zugewandten Seite der Halterung 4 ist eine Bedieneinheit 10 für die Hauptfunktionen der Röntgendiagnostikanlage und der Patientenlagerung befestigt.

Sie kann ebenfalls um die Längsachse 7 der Halterung 4 schwenkbar gelagert sein, so daß sie, wenn sie nicht benötigt wird, aus dem Untersuchungsbereich herausgeklappt werden kann. Mit dieser Bedieneinheit 10 kann die Untersuchungsperson vorteilhaft alle notwendigen Bedienelemente zum Einstellen der Röntgendiagnostikanlage bequem erreichen und bedienen.

Besonders vorteilhaft ist, wenn die Bedieneinheit 10 kabellos arbeitet. Hierdurch wird die Stolpergefahr für die sich im Untersuchungsbereich befindenden Personen erheblich reduziert. Außerdem werden Behinderungen durch herumliegende Kabel beim Verfahren von Geräten im Untersuchungsbereich ausgeschlossen.

Ein weiterer Vorteil ergibt sich, wenn ein Fußschalter 11 für beispielsweise die Funktionen Durchleuchtung und Aufnahmeauslösung auf dem Fahrgestell 2 montiert ist, der ebenfalls kabellos arbeitet. Hierdurch kann die Strahlenschutzvorrichtung ohne die Behinderung durch Verbindungskabel frei im Untersuchungsbereich verfahren werden.

Vorteil der erfindungsgemäßen Strahlenschutzvorrichtung ist, daß die Untersuchungsperson keinen schweren Bleigummimantel tragen muß, und daß sie dennoch einen Ganzkörperstrahlenschutz erhält.

## Ansprüche

1. Strahlenschutzvorrichung (1) mit einem, eine Strahlenschutzwand tragenden Wagen (2), der frei verfahrbar ist und mit einer Halterung (4) für zwei Wandelemente (5, 6), wobei die Halterung (4) höhenverstellbar auf dem Wagen (2) montiert ist, wobei das erste Wandelement (5) unterhalb des zweiten, durchsichtigen Wandelementes (6) angeordnet ist und wobei das zweite Wandelement (6) um eine horizontale Achse (7) schwenkbar ist.

2. Strahlenschutzvorrichtung nach Anspruch 1, **dadurch gekennzeichnet**, daß die Halterung (4) als Balken mit horizontaler Achse (7) annähernd parallel zum Boden angeordnet ist.

3. Strahlenschutzvorrichtinng nach Anspruch 1 oder 2, **dadurch gekennzeichnet**, daß das erste Wandelement (5) aus Bleigummi hergestellt ist und kurz vor dem Bodenniveau endet und daß das zweite Wandelement (6) aus Bleiglas hergestellt ist und über die Kopfhöhe einer durchschnittlichen Untersuchungsperson reicht.

4. Strahlenschutzvorrichtinng nach einem der Ansprüche 1 bis 3, **dadurch gekennzeichnet**, daß jeweils im Bereich der Halterung (4) höhenverstellbare Stützschalen (8) zum Stützen der Ellenbogen der Untersuchungsperson vorgesehen sind.

5. Strahlenschutzvorrichtinng nach einem der Ansprüche 1 bis 4, **dadurch gekennzeichnet**, daß im Bereich der Halterung (4) eine Bedieneinheit (10) für die Rontgendiagnostikanlage und die Patientenlagerung angeordnet ist.

6. Strahlenschutzvorrichtung nach Anspruch 1 bis 5, **dadurch gekennzeichnet**, daß im Bereich der Halterung (4) eine Haltevorrichtung (9) für Bleihandschuhe vorgesehen ist.

7. Strahlenschutzvorrichtung nach einem der Ansprüche 1 bis 6, **dadurch gekennzeichnet**, daß der Wagen (2) als auf dem Boden verfahrbares Fahrgestell ausgebildet ist, an dem ein Fußschalter (11) für die Röntgendiagnostikanlage vorgesehen ist.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | DE-U-1 958 862  (H. WÄLISCHMILLER) * Seite 4, Zeilen 6-19; Schutzanspruch 1; Figuren 1,2 * | 1,3 | A 61 B   6/10 |
| A | | 7 | |
| | --- | | |
| Y | US-A-3 984 696  (C. COLLICA et al.) * Zusammenfassung; Spalte 2, Zeilen 26-67; Figuren 1-4 * | 1,3 | |
| A | | 2 | |
| | --- | | |
| A | FR-A-1 318 217  (SIEMENS-REINIGER-WERKE AG) * Seite 1, rechte Spalte, Zeile 17 - Seite 2, linke Spalte, Zeile 48; Figur 1 * | 1,5-7 | |
| | --- | | |
| A | GB-A-  490 480  (O.C. HARTRIDGE) * Seite 3, Zeilen 2-14,51-96; Seite 4, Zeilen 8-19,56-62; Figuren 1-3 * | 1-3,6,7 | |
| | --- | | |
| A | DE-C-  894 916  (R. WITTMOSER) * Seite 2, Zeilen 60-68,84-93; Figur 1 * | 7 | RECHERCHIERTE SACHGEBIETE (Int. Cl.4) |
| | ----- | | A 61 B |

Der vorliegende Recherchenbericht wurde für alle Patentansprüche erstellt

| Recherchenort | Abschlußdatum der Recherche | Prüfer |
|---|---|---|
| DEN HAAG | 14-08-1989 | RIEB K.D. |